# EUROPÄISCHE PATENTSCHRIFT

(11) **EP 1 525 461 B1**
(45) Veröffentlichungstag und Bekanntmachung des Hinweises auf die Patenterteilung: **10.09.2008**
(21) Anmeldenummer: 03783986.7
(22) Anmeldetag: 25.06.2003
(51) Int. Cl.: G01N 27/28, G01N 33/12

(54) **PH-MESSVORRICHTUNG MIT AXIAL BEWEGLICHER EINSTECHELEKTRODE**
PH MEASURING DEVICE WITH AN AXIALLY MOVABLE PENETRATING ELECTRODE
DISPOSITIF DE MESURE DE PH EQUIPE D'UNE ELECTRODE DE PENETRATION MOBILE AXIALEMENT

(30) Priorität: 25.07.2002 DE 10233901
(43) Veröffentlichungstag der Anmeldung: 27.04.2005
(73) Patentinhaber: Testo AG, 75853 Lenzkirch (DE)
(72) Erfinder: DERR, Andreas, 79793 Wutöschingen (DE)
(74) Vertreter: Schmuckermaier, Bernhard
(86) Internationale Anmeldenummer: PCT/EP2003/006714
(87) Internationale Veröffentlichungsnummer: WO 2004/015407

(56) Entgegenhaltungen:
- EP-A- 0 399 101
- EP-A- 0 753 737
- EP-A- 0 964 060
- DE-A- 10 004 583
- FR-A- 2 618 902

## Beschreibung

Die vorliegende Erfindung betrifft eine Messvorrichtung mit Einstechelektrode der im Oberbegriff des Patentanspruchs 1 genannten Art. Die Erfindung betrifft ferner ein Messgerät, ein Verfahren zur Herstellung und eine Verwendung einer Messvorrichtung.

Eine gattungsgemässe Messvorrichtung ist aus der DE 38 14 634A1, der US 4,252, 124, und der US 4,218, 299 bekannt.

Derartige Messvorrichtung dienen beispielsweise der Messung des pH-Wertes von Lebensmitteln, wie z. B. von Fleisch. Üblicherweise ist zwischen der erste Elektrode und der Ummantelung eine Kammer gebildet, in der eine, beispielsweise aus einem Gel ausgebildete, zweite Elektrode untergebracht ist. Beide Elektroden sind zur Ermittlung des pH-Wertes einer zwischen die Elektroden eingedrungenen Flüssigkeit des Messguts an eine Auswerteschaltung angeschlossen.

Erste Elektroden zur pH-Messung sind üblicherweise aus Glas oder weisen Glas als Ummantelung auf. Zur Messung wird die Messvorrichtung mit der Glaselektrode mit grosser Kraft in das Messgut eingetrieben. Dabei wird die Elektrode in axialer Richtung, also der Einstechrichtung, einer erheblichen mechanischen Belastung unterworfen, beispielsweise durch einen Stoss der Elektrode auf einen Knochen. Aufgrund derSprödigkeit von Glas sind diese Glaselektroden nur wenig belastbar, was beispielsweise beim Verkanten, beim Einstechen oder Herausziehen der Glaselektrode oder beim Herunterfallen, häufig zum Bruch führen kann.

Zur Vermeidung dieses Problems sind Messgeräte bekannt, bei denen die Glaselektrode bzw. das die Glaselektrode umgebende Gel zunächst von einer Glasummantelung und dann von einer Metall- oder Kunststoffhülse umhüllt werden. Dadurch wird zwar die Belastbarkeit der Glaselektrode senkrecht zur Axialrichtung etwas verbessert, geringste Verbiegungen der Ummantelung, die direkt auf die Glaselektrode übertragen werden, führen aber dennoch zum Bruch der Glaselektrode und/oder der das Gel umgebenden Glashülle.

Aufgrund dieser doppelten Ummantelung aus Glas und Kunststoff ist das Messgerät im Bereich der Sondenspitze unvorteilhaft dick, wodurch beim Messen größere Löcher in dem Messgut hervorgerufen werden. Zudem sind solche Messgeräte mit dicken Messsonden für Messungen in Behältern mit kleinen Einführungsöffnungen, wie zum Beispiel Flaschen, Reagenzgläsern und dergleichen, ungeeignet.

In der DE 100 04 583 A1 ist eine weitere gattungsgemäße Messvorrichtung mit Einstechelektrode beschrieben. Zur Vermeidung eines Bruchs der Glaselektrode ist die langgestreckte Glaselektrode schwenkbar gelagert. Dadurch kann die Glaselektrode im Falle einer Querbelastung senkrecht zu deren Axialrichtung dieser Belastung bis zu einem bestimmten Grad ausweichen, wodurch in diesem Falle ein Bruch der Glaselektrode weitestgehend vermieden wird. Problematisch ist nach wie vor, wenn die Glaselektrode zusätzlich oder ausschließlich einer Kraft in Axialrichtung der Glaselektrode unterworfen wird, beispielsweise wenn sie direkt auf einen harten Knochen trifft oder zu Boden fällt. In diesem Falle besteht nach wie vor die Gefahr eines Glasbruches.

Der vorliegenden Erfindung liegt daher die Aufgabe zugrunde, eine gegenüber Belastungen in Axialrichtung der Einstechelektrode robuster ausgebildete Messvorrichtung bereit zu stellen. Ferner soll ein Verfahren und eine Verwendung angegeben werden.

Die vorrichtungsbezogene Aufgabe wird erfindungsgemäß durch eine Messvorrichtung mit den Merkmalen des Patentanspruchs 1 und ein tragbares Messgerät mit den Merkmalen des Patentanspruchs 23 gelöst. Die verfahrensbezogene Aufgabe wird durch ein Verfahren gemäß Patentanspruchs 25, die verwendungsbezogene Aufgabe durch eine Verwendung gemäß Patentanspruch 27 gelöst.

Danach ist die langgestreckte erste Elektrode der Messvorrichtung in ihrer Axialrichtung beweglich gelagert und kann so bei einer Belastung bzw. einem Stoß in Richtung ihrer Achse dieser Belastung bis zu einem bestimmten Grad in das Gehäuse der Messvorrichtung hinein ausweichen. Durch diese stoßdämpfende Charakteristik kann ein Bruch der vorzugsweise als Glaselektrode ausgebildeten ersten Elektrode, weitestgehend vermieden werden. Axial bewegliche Elektroden sind für nicht gattungsgemäße Messvorrichtungen bekannt, z.B.EP0753737.

Die erste Elektrode ist vorteilhafterweise formschlüssig in einer Aufnahmevorrichtung eingebettet. Die Aufnahmevorrichtung kann beispielsweise als Aussparung ausgebildet sein, die an ihren Seitenwänden Dichtvorrichtungen zum Abdichten und Fixieren der ersten Elektrode aufweist.

Gemäß einer ersten Ausführungsform der Erfindung besteht die Aufnahmevorrichtung aus einem elastischen Material, beispielsweise Gummi, welches bei einem Druck der ersten Elektrode auf die Aufnahmevorrichtung in Axialrichtung nachgibt.

Zusätzlich oder alternativ kann die Aufnahmevorrichtung ein Dämpfungselement aufweisen, das bei einem Druck der Elektrode in Axialrichtung nachgibt. Das Dämpfungselement kann vorteilhafterweise als Gummipuffer oder als mechanische Feder ausgebildet sein. Es könnte jedoch auch ein pneumatisch oder hydraulisch ausgebildetes Dämpfungselement vorgesehen sein.

In einer weiteren Ausführungsform der Erfindung ist die erste Elektrode in eine Aufnahmevorrichtung, die Bestandteil der vorteilhafterweise elastisch ausgebildeten Bodenplatte des Gehäuses ist, eingesetzt. Zusätzlich oder alternativ kann die Bodenplatte membranartig derart ausgebildet sein, dass sie Falzabschnitte aufweist. Bei einer Belastung der ersten Elektrode dehnen sich die Falzabschnitte senkrecht zur Axialrichtung aus, wodurch die so ausgebildete Bodenplatte nachgibt und ebenfalls dämpfend wirkt.

In einer vorteilhaften Ausgestaltung sind die Elektroden mit Kontaktstiften, die durch die Bodenplatte hindurchragen, elektrisch leitend verbunden. An der äußeren Fläche der Bodenplatte sind ferner Ösen vorgesehen. Die Kontaktstifte sind hier so abgebogen, dass sie in diese Ösen der Bodenplatte eingeschlauft werden können. Die so abgebogenen und in die Ösen eingeschlauften Kontaktstifte bilden den jeweiligen Elektroden zugeordnete Kontaktflächen, über die die Messvorrichtung von außen kontaktiert werden kann.

Die Elektroden können einerseits fest an der Bodenplatte bzw. dem Gehäuse angeklebt oder dort angeschweißt sein. Alternativ wäre es insbesondere bei Verwendung einer Aufnahmevorrichtung auch von Vorteil, wenn die Elektroden formschlüssig mit ihrem einen Ende in eine Aussparung der Aufnahmevorrichtung eingebettet sind. Zu diesem Zwecke weist die Aufnahmevorrichtung typischerweise Dicht- und Einrastvorrichtungen zur Fixierung der ersten Elektrode innerhalb der Aufnahmevorrichtung auf.

Das Messmodul besteht aus einem Gehäuse, welches die erste Elektrode fest umschließt und nach außen hin abdichtet. Das Innere des Gehäuses definiert eine Kammer, in der eine Elektrodenflüssigkeit, die die erste und/oder die zweite Elektrode umgibt, eingefüllt ist. Als Elektrodenflüssigkeit ist vorteilhafterweise eine Polymerprotolyt-Flüssigkeit oder -Gel vorgesehen. Ein Messmodul mit Polymerprotolyt-Aufbau hat den Vorteil, dass der Messbereich um problematische Messstellen, beispielsweise stark belastete Abwässer, eiweißhaltige Flüssigkeiten, etc., erweitert werden kann. Zudem können Fehlpotentiale und Ausfälle bei Messungen in solchen Flüssigkeiten verhindert werden. Ein Verstopfen oder eine Beschädigung bei solchen Messungen unter Verwendung herkömmlicher pH-Messmodule, die als Elektrolyt eine Silber gesättigte Kaliumchloridlösung enthalten, wird damit verhindert.

Die Messsonde besteht in einer vorteilhaften Ausgestaltung aus einem Glasröhrchen, in dem die erste Elektrode und eine Elektrolytflüssigkeit vorgesehen sind. Bei einer Belastung der Messsonde wird lediglich das Glasröhrchen in Axialrichtung verschoben, während die erste Elektrode, die beispielsweise fest an dem Gehäuse befestigt ist, nicht bewegt wird. In einer weiteren vorteilhaften Ausgestaltung weist die Ummantelung an der Messspitze der Messsonde ebenfalls eine Hülse auf, die gegen die Messspitze verschiebbar angeordnet ist. Auf diese Weise kann die Messspitze der Messsonde zusätzlich geschützt werden.

In einer vorteilhaften Ausgestaltung weist die erfindungsgemäße Messvorrichtung in Richtung zur Messspitze einen schlanken Schaft auf, der gegenüber den übrigen Bereichen des Gehäuses einen sehr viel geringeren Durchmesser aufweist. Speziell im Laborbereich sind Anwender von Messvorrichtungen schlanke Handhabungsgeräte, wie z. B. Dosierpipetten, gewohnt. Aufgrund dieses schlanken Designs der Messvorrichtung ist diese auch für Messungen in Behältern mit kleinen Einführungsöffnungen, z. B. Flaschen, Reagenzgläsern oder sonstigen Behältern, geeignet. Die kleine Spitze des Messmoduls ermöglicht zudem eine Aufbewahrung der Messsonde in einem Gürtelhalter mit integriertem Behälter, in dem eine Aufbewahrungslösung für die Elektroden des pH-Messmoduls eingefüllt ist.

In einer typischen Ausgestaltung besteht die erste Elektrode und/oder deren Schutzhülse zumindest teilweise aus Glas.

In einer weiteren, sehr vorteilhaften Ausgestaltung ist die erste Elektrode schwenkbar gelagert. Die erste Elektrode der Messvorrichtung kann so bei Belastung senkrecht zur Axialrichtung dieser Belastung bis zu einem bestimmten Grad ausweichen, wodurch ein Bruch der typischerweise als Glaselektrode ausgebildeten ersten Elektrode zusätzlich vermieden werden kann. Zu diesem Zweck weist die erste Elektrode Schwenkmittel, z.B. ein Gelenk, auf. Hinsichtlich der verschiedenartigen Ausgestaltung und Funktionsweise dieser Schwenkmittel wird auf die eingangs genannte DE 100 04 583 A1 verwiesen, die hiermit vollinhaltlich mit in die vorliegende Patentanmeldung mit einbezogen wird.

Die Ummantelung bzw. das Gehäuse des Messmoduls besteht zumindest zum Teil aus einem SAN- oder ABS-enthaltenden Werkstoff. Es handelt sich dabei nicht um Elastomere, sondern um Kunststoffe, die vorteilhafterweise im bestimmten Bereich elastisch sind.

In einer sehr vorteilhaften Ausgestaltung ist die Messvorrichtung modular ausgebildet. Dieses Messmodul kann somit als Bestandteil eines tragbaren pH-Messgerätes auf dieses aufgesteckt werden. Aufgrund dieser modularen Funktionalität des Messmoduls kann es jederzeit ausgewechselt werden. Bei einem funktionsunfähigen pH-Messmodul muss somit lediglich das Messmodul ausgewechselt werden, während das eigentliche Messgerät weiterhin verwendet werden kann.

Besonders vorteilhaft ist die erfindungsgemäße Messvorrichtung bzw. das erfindungsgemäße Messgerät bei der Messung des pH-Wertes in Lebensmitteln, von eiweißhaltigen Flüssigkeiten oder Abwässern. Dies ist erst durch die Verwendung einer Polymerprotolyt-Lösung in der Kammer der Messvorrichtung möglich.

Weitere vorteilhafte Ausgestaltungen und Weiterbildungen der Erfindung sind den Unteransprüchen sowie der Beschreibung unter Bezugnahme auf die Zeichnung entnehmbar.

Die Erfindung wird nachfolgend anhand der in den Figuren der Zeichnung angegebenen Ausführungsbeispiele näher erläutert. Es zeigt dabei:
- Figur 1: eine schematische Querschnittsdarstellung einer erfindungsgemäßen Messvorrichtung zur Darstellung des Grundprinzips der Erfindung;
- Figur 2: ein erstes Ausführungsbeispiel eines erfindungsgemäßen Messmoduls;
- Figur 3: ein zweites Ausführungsbeispiel eines erfindungsgemäßen Messmoduls;
- Figur 4: ein drittes Ausführungsbeispiel eines erfindungsgemäßen Messmoduls;
- Figur 5: ein viertes Ausführungsbeispiel eines erfindungsgemäßen Messmoduls im unbelasteten (Fig. 5a) und belasteten Zustand (Fig. 5b);
- Figur 6: das Gehäuse eines erfindungsgemäßen Messmoduls;
- Figur 7: ein fünftes Ausführungsbeispiel eines erfindungsgemäßen Messmoduls;
- Figur 8: ein sechstes Ausführungsbeispiel eines erfindungsgemäßen Messmoduls;
- Figur 9: eine Detaildarstellung einer Bodenplatte;
- Figur 10: ein vorteilhaftes Verfahren zur Herstellung eines Messmoduls entsprechend Figur 6;
- Figur 11: eine Querschnittsdarstellung eines Messgeräts mit einem modulartig aufgesteckten, erfindungsgemäßen Messmodul.

In allen Figuren der Zeichnung sind gleiche beziehungsweise funktionsgleiche Elemente - sofern nichts anderes angegeben ist - mit gleichen Bezugszeichen versehen worden.

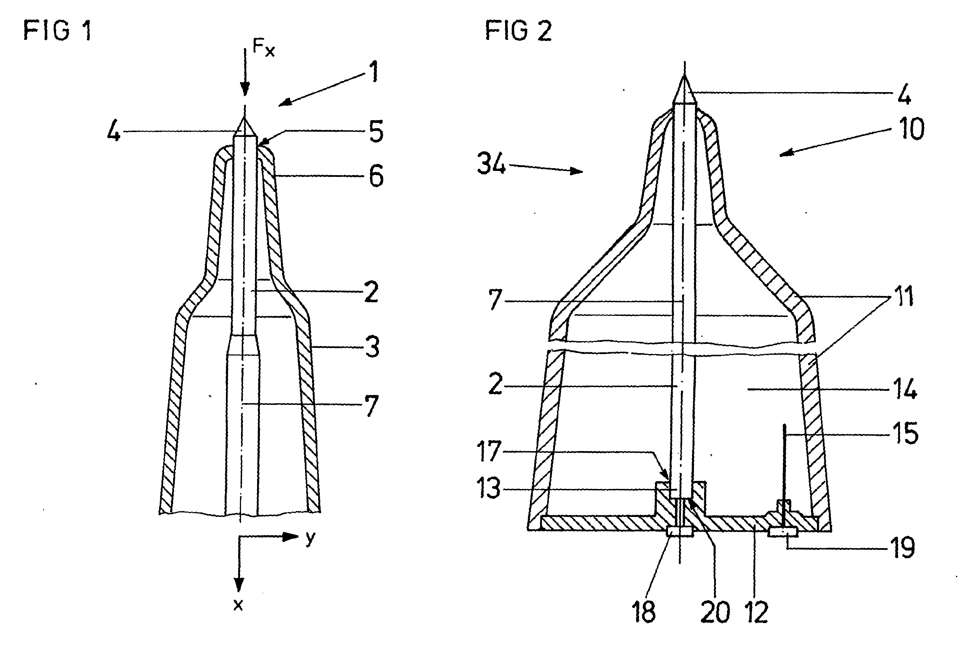

Figur 1 zeigt in einer schematischen Querschnittsdarstellung einen Ausschnitt einer erfindungsgemäßen Messvorrichtung, die das Prinzip der vorliegenden Erfindung in allgemeiner Weise darstellt.

In Figur 1 ist mit Bezugszeichen 1 ein Ausschnitt einer erfindungsgemäßen Messvorrichtung bezeichnet. Die Messvorrichtung 1 weist eine langgestreckte erste Elektrode 2 und ein die erste Elektrode 2 wenigstens teilweise umgebendes Gehäuse 3 auf. Eine Messspitze 4 der ersten Elektrode 2 ragt hier aus einer eigens dafür vorgesehenen Öffnung 5 am oberen Ende 6 des Gehäuses 3 heraus. Die typischerweise zylinderförmig ausgebildete erste Elektrode 2 besitzt eine Längsachse 7, die eine Axialrichtung X definiert. Erfindungsgemäß ist die erste Elektrode 2 bei einer Beaufschlagung mit einer Kraft Fₓ in Axialrichtung X in diese Richtung relativ zum Gehäuse 3 bewegbar.

Anhand der Figuren 2 bis 7 werden nachfolgend fünf Ausführungsbeispiele einer erfindungsgemäßen Messvorrichtung dargestellt. Die erfindungsgemäße Messvorrichtung ist hier als auswechselbares Messmodul 10 ausgebildet. Das Messmodul 10 ist als pH-Messmodul zur Messung des pH-Wertes von Flüssigkeiten, Lebensmitteln, Abwässern und dergleichen ausgebildet.

Das Gehäuse 3 weist eine Ummantelung 11 sowie eine Bodenplatte (Trägerplatte) 12 auf. Die Ummantelung 11 besteht vorzugsweise aus einem mehr oder weniger elastischen Kunststoff. Die erste Elektrode 2 ist an ihrem einen Ende 13 mit der Bodenplatte 12 verbunden, während ihr anderes Ende, d.h. die Messspitze 4, aus der Ummantelung 11 bzw. dem Gehäuse 3 herausragt. Die erste Elektrode 2 besteht vorzugsweise aus Glas oder weist eine Ummantelung aus Glas auf und dient als Messsonde 34 der Bestimmung des pH-Wertes eines Messgutes. Die Bereiche zwischen der ersten Elektrode 2 und dem Gehäuse 3 definieren eine Kammer 14. Die Messvorrichtung weist ferner eine zweite Elektrode 15 auf, die hier als Silberelektrode ausgebildet ist und die von der Bodenplatte 12 in die Kammer 14 hineinragt. Die Kammer 14 ist vorteilhafterweise durch eine Polymerprotolyt-Lösung gefüllt.

Das Gehäuse 3 ist nach außen hin, mit Ausnahme eines Einlasses für die Messflüssigkeit, dicht verschlossen. Zu diesem Zwecke ist die Ummantelung 11 beispielsweise mittels einer Dichtvorrichtung, einer Klebeschicht, einer Schweißnaht oder dergleichen nach außen hin dichtend mit der Bodenplatte 12 verbunden.

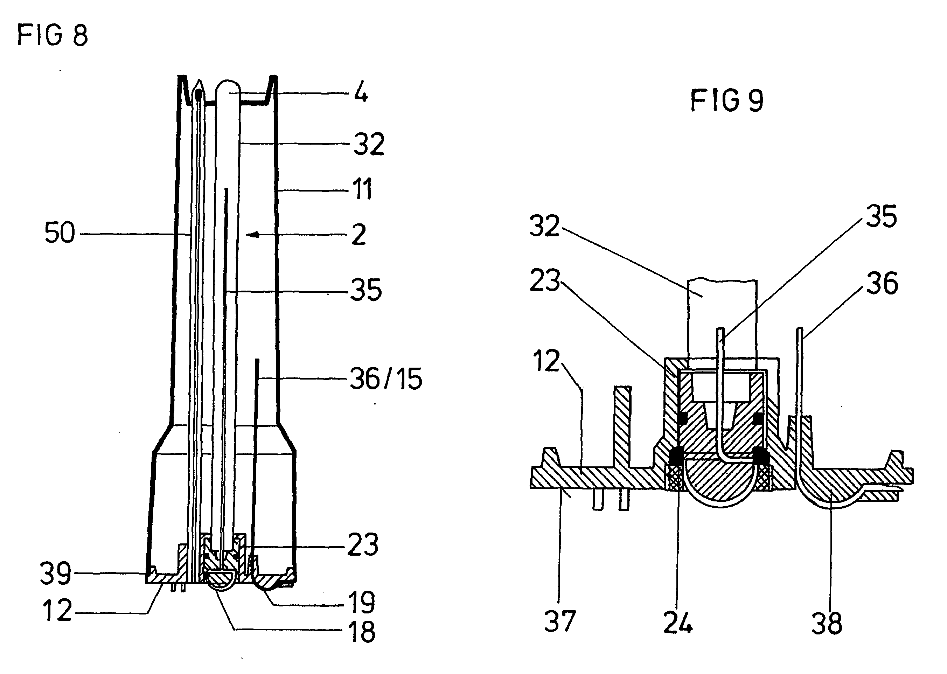

In Figur 2 besteht die mit der ersten Elektrode 2 verbundene Bodenplatte 12 aus einem elastischen Material. Die Bodenplatte 12 weist eine Aussparung 17 auf, die der Aufnahme der ersten Elektrode 2 dient. Dabei ist das Ende 13 der ersten Elektrode 2 mehr oder weniger formschlüssig in diese Aussparung 17 eingefügt und durch geeignete Mittel, beispielsweise Dichtringe, Einrastvorrichtungen, Klebstoffe, etc., fixiert. Die Bodenplatte 12 weist ferner an ihrer Außenseite Kontaktflächen 18, 19, beispielsweise Goldkontakte, auf. Die erste und zweite Elektrode 2, 15 sind über geeignete Mittel, die nachfolgend noch anhand von Figur 8 erläutert werden, mit diesen Kontakten 18, 19 elektrisch leitend verbunden.

Die erste Elektrode 2 liegt fest auf dem Boden 20 der Aussparung 17 auf und ist somit starr mit der Bodenplatte 12 gekoppelt. Wird die erste Elektrode 2 mit einer Kraft Fₓ in Axialrichtung X beaufschlagt, dann gibt die fest mit der ersten Elektrode 2 gekoppelte Bodenplatte 12 aufgrund ihrer Materialeigenschaften membranartig nach und dämpft auf diese Weise den durch die Kraft Fₓ hervorgerufenen Stoß ab. Die Glaselektrode 2 wird dadurch geringfügig in Axialrichtung X in das Gehäuse 3 geschoben, wodurch eine Beschädigung bzw. eine Zerstörung der Glaselektrode 2 verhindert wird. Das Ausführungsbeispiel in Figur 2 zeigt die einfachste Ausführungsvariante zur Realisierung eines erfindungsgemäßen Messmoduls 10, welches insbesondere montagetechnisch und aus Kostengründen besonders vorteilhaft ist.

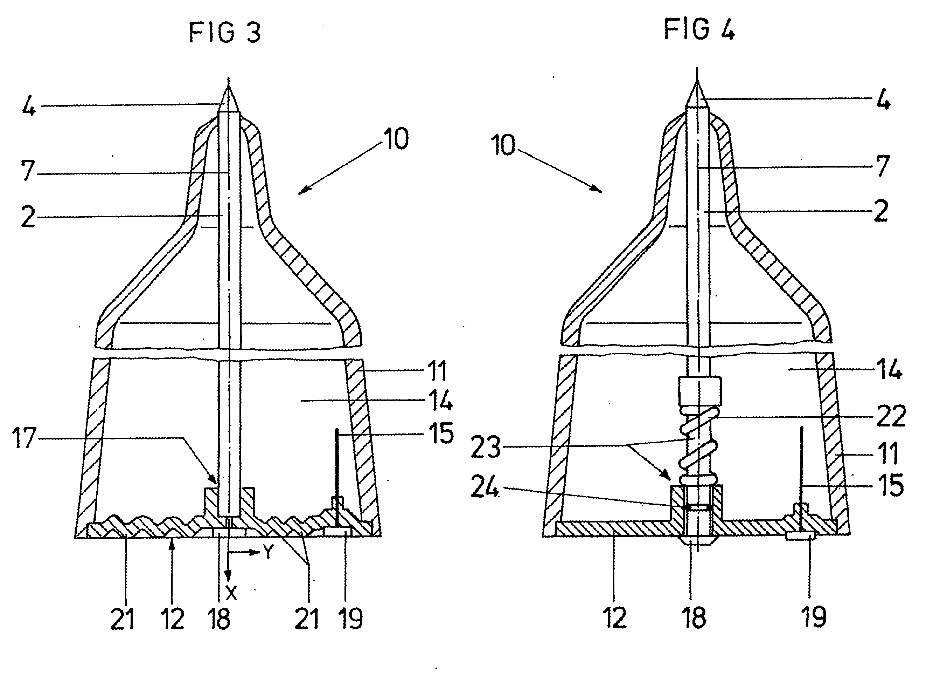

Im Unterschied zu der Variante in Figur 2 weist das Messmodul 10 in Figur 3 eine membranartig ausgeformte Bodenplatte 12 auf. Die Bodenplatte 12 weist hier gefalzte Abschnitte 21 auf, die im Falle eines Stoßes Fₓ in Axialrichtung X durch Ausdehnung senkrecht zur Axialrichtung X, also in Y-Richtung, diesen Stoß dämpfen.

Im Unterschied dazu ist im Ausführungsbeispiel in Figur 4 eine Feder 22 vorgesehen. Diese Feder 22 ist zwischen Glaselektrode 2 und Bodenplatte 12 angeordnet. Die Feder 22 wird durch eine eigens dafür vorgesehene Aufnahmevorrichtung 23 in der Bodenplatte 12 fixiert und mittels eines Ohrringes 24 nach außen hin abgedichtet.

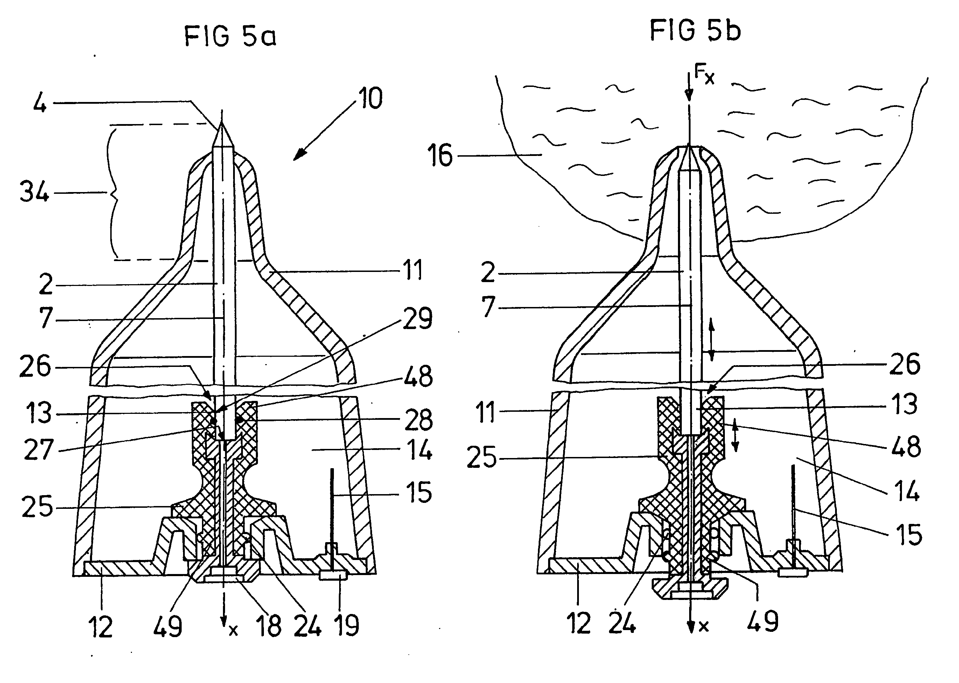

Im Ausführungsbeispiel der Figuren 5a, 5b ist ein Gummipuffer 25 vorgesehen. Der Gummipuffer 25 besteht aus einem elastischen, kompressionsfähigen Material, z. B. aus Gummi oder aus einem schwammartigen Material. Der Gummipuffer 25 ragt durch eine in der Bodenplatte 12 eigens dafür vorgesehene Öffnung in die Kammer 14 hinein. An seinem oberen, in das Kammerinnere 14 hineinragende Ende 48 weist der Gummipuffer 25 eine Aussparung 26 auf, in die das Ende 13 der Glaselektrode 2 formschlüssig eingefügt ist. Dieses Ende 13 liegt am Boden 27 dieser Aussparung 26 auf und wird mittels umlaufender Dichtlippen 28, die an den Seitenflächen 29 der Aussparung 26 vorgesehen sind, in der Aussparung 26 positioniert. An der anderen Seite 49 des Gummipuffers 25, an der dieser durch eine Öffnung der Bodenplatte 12 hindurch ragt, ist ein umlaufender O-Ringe 24 zur Abdichtung nach außen vorgesehen.

Nachfolgend wird die Funktionsweise des Messmoduls 10 anhand der Figuren 5a, 5b erläutert:

Zur Messung wird die Messsonde 34 des Messmoduls 10 in das Messgut 16 eingebracht, wie dies beispielsweise in Figur 5b dargestellt ist. Mittels geeigneter, hier nicht näher dargestellter Maßnahmen, beispielsweise einem Diaphragma, gelangt Flüssigkeit des Messguts 16 in den Bereich zwischen die erste Elektrode 2 und die zweite Elektrode 15. Zur Erleichterung des Einbringens in das Messgut 16 weist die Messsonde 34 an ihrem, aus dem Messmodul 10 herausragenden Ende eine Einstechspitze 4 auf. Durch das Einstechen in das Messgut 16 wird eine Kraft Fₓ in Axialrichtung X auf die Glaselektrode 2 ausgeübt. Die Dämpfung des als Gummipuffer 25 ausgebildeten Dämpfungselementes ist so ausgelegt, dass durch das bloße Einstechen in das zu messende Gut 16, beispielsweise Fleisch, die Glaselektrode 2 nicht bzw. nur unwesentlich in Axialrichtung X in das Gehäuse 3 hinein verschoben wird. Trifft die Glaselektrode 2 hingegen in dem Messgut 16 auf einen harten Gegenstand, beispielsweise einen Knochen, dann ist die von diesem Knochen auf die Glaselektrode 2 ausgeübte axiale Kraft Fₓ so groß, dass die Glaselektrode 2 in Axialrichtung X in das Gehäuse 3 geringfügig hinein verschoben wird. Die Elastizität des Gummipuffers 25 ist so bemessen, dass die Kraft Fₓ ausreicht, um den Gummipuffer 25 zu verformen bzw. zu komprimieren. Auf Grund der Verformung bzw. Kompression des Gummipuffers 25 wird die Glaselektrode 2 in das Gehäuse 3 gedrückt und auf diese Weise vor einer Beschädigung bzw. Zerstörung geschützt. Der gleiche Effekt tritt ein, wenn das Messmodul 10 mit seiner Messspitze 4 einem plötzlichen Stoß, wie er beispielsweise beim Herunterfallen auf den Boden vorkommt, unterworfen wird.

In den Ausführungsbeispielen gemäß der Figuren 2 und 3 ist eine Bodenplatte 12, im Ausführungsbeispiel in Figur 4 eine Feder 22 und im Ausführungsbeispiel in Figur 5 ein Gummipuffer 25 als Dämpfungselement vorgesehen. In den Ausführungsbeispielen gemäß der Figuren 4 bis 5 besteht die Bodenplatte 12 jeweils aus einem formstabilen, festen Material, welches sich bei einem Kraftstoß Fₓ nicht verformt. Jedoch wäre es auch denkbar für die Bodenplatte 12 zusätzlich ein flexibles, elastisches Material wie im Beispiel in Figur 2 zu verwenden, da dadurch der Dämpfungseffekt weiter verbessert wird.

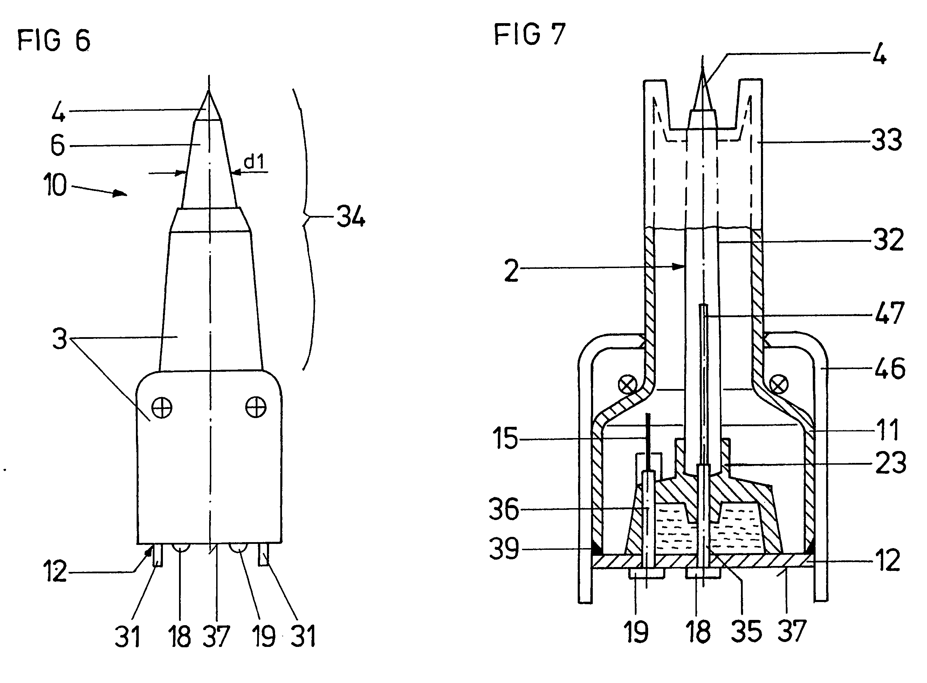

Figur 6 zeigt ein vollständiges Gehäuse 3 eines modular aufgebauten Messmoduls 10. Dieses Messmodul 10 kann beispielsweise entsprechend den Figuren 2 bis 5 ausgestaltet sein. An der Unterseite 37 der Bodenplatte 12 sind Kontaktflächen 18, 19 vorgesehen. An dieser Seite 37 weist die Bodenplatte 12 ferner Steckverbindungen 31 auf, mittels denen das Messmodul 10 auf ein in Figur 6 nicht dargestelltes Messgerät aufsteckbar ist. Das Messmodul 10 weist eine Messsonde 34 auf. Diese Messsonde 34 verjüngt sich in Richtung zur Messspitze 4, so dass am oberen Ende 6 des Gehäuses 3 die Messsonde 34 einen sehr viel geringeren Durchmesser d1 aufweist als im unteren, zur Bodenplatte 12 gewandten Bereich. Auf diese Weise lässt sich die Messsonde 34 auch in Flaschen, Reagenzgläsern oder sonstigen Behältern mit geringerem Öffnungs-Durchmesser einführen.

In den Ausführungsbeispielen gemäß der Figuren 2 bis 5 ist eine als Glaselektrode ausgebildete erste Elektrode 2 dargestellt. Alternativ wäre es entsprechend Figur 7 auch denkbar, wenn die Messsonde 34 aus einem Silberstift 47 sowie ein diesen Silberstift 47 umgebendes Glasröhrchen 32 besteht. Zwischen Silberstift 47 und Glasröhrchen 32 ist typischerweise eine Elektrolytflüssigkeit eingefüllt. Zusätzlich oder alternativ kann das Gehäuse 3 an seinem oberen Ende 6 eine Schutzhülle 46 aufweisen, die das Gehäuse 3 des Messmoduls 10 nach außen hin, beispielsweise gegen mechanische Belastung, Feuchtigkeit oder dergleichen, schützt. Das von oben in das Messmodul 10 eingeschobene Glasröhrchen 32 ist im Unterschied zu den Ausführungsbeispielen der Figuren 2 bis 5 in seiner Verankerung in der Aufnahmevorrichtung 23 angeklebt ist. Zusätzlich oder alternativ kann das Gehäuse 3 an seinem oberen Ende 6 Schutzstege 33 aufweisen, die die Messsonde 34 zusätzlich schützt (Figuren 7, 8).

Aufgrund dieser Schutzstege weist die Messsonde 34 gemäß dem Ausführungsbeispiel in Figur 8 nicht mehr die für ein Einstechen in das Messgut 16 vorteilhafte, spitz ausgebildete-Messspitze 4 auf, sie ist dafür aber optimal geschützt. Das Messgerät 10 ist hier besonders gut für Messungen in Flüssigkeiten geeignet und findet daher vorteilhaft Verwendung als Labormessgerät. Die Messspitze 4 ist aus diesem Grunde typischerweise abgerundet . Zusätzlich ist in Figur 8 ein zur Messspitze hin spitz zulaufendes Röhrchen 50 vorgesehen, welches einerends mit der Bodenplatte 12 fest verbunden ist und andererends aus dem Gehäuse 3 herausragt. Dieses spitze Röhrchen 50, welches vorteilhafterweise aus rostfreien Stahl besteht, enthält einen Temperatursensor und dient der Temperaturbestimmung des Messguts 16.

Figur 9 zeigt in einer Detaildarstellung die Bodenplatten 12 sowie die darin verankerten Elektroden 2, 15. Die Elektroden 2, 15 sind starr mit der Bodenplatte 12 verbunden, sei es durch Ankleben der Elektrode in die Aufnahmevorrichtung 23 entsprechend der Figuren 7 bis 9 oder sei es durch formschlüssiges Einstecken in Aufnahmevorrichtungen 23 entsprechend den Figuren 2 bis 5. Ein jeweils mit den Elektroden 2, 15 verbundener, elektrisch leitender Stift 35, 36 ragt durch die Bodenplatte 12 hindurch. An der Unterseite 37 der Bodenplatte 12 weist diese spritzgegossene Ösen 38 auf. Um definierte Kontaktflächen 18, 19 zu erhalten, wird der eine Stift 36 in eine Öse 38 eingeschlauft, wohingegen der andere Stift 35 geeignet um die in der Bodenplatte 12 vorgesehene Aufnahmevorrichtung 23 gebogen wird.

Die in der Bodenplatte 12 umspritzte Elektrode 15 bildet mit dem in der Kammer 14 eingefüllten Polymerprotolyt-Gel, welches sich zwischen dem Gehäuse 3 und der Messsonde 2 befindet, die zweite Elektrode 15.

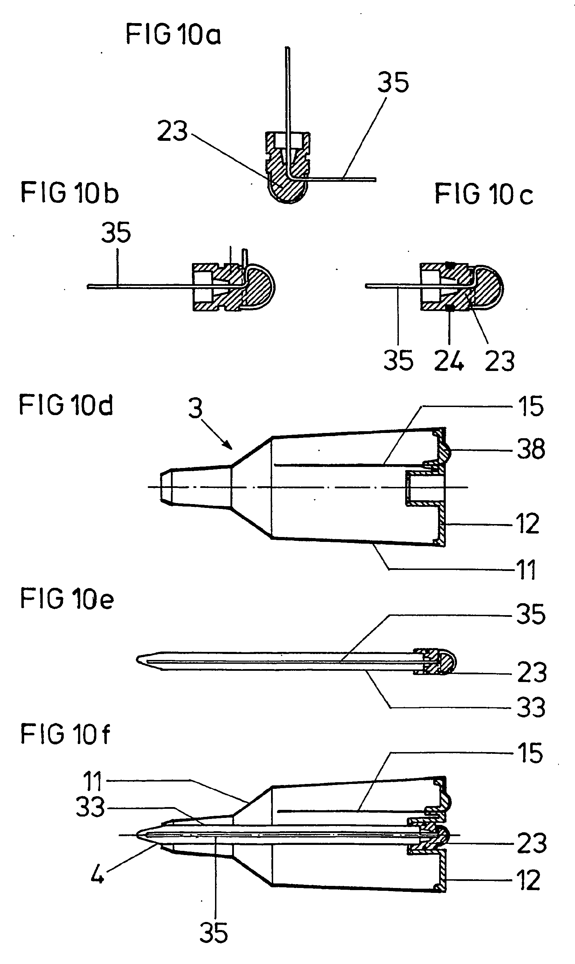

Nachfolgend wird anhand von Figur 10 ein vorteilhaftes Verfahren zur Herstellung eines Messmoduls 10 beschrieben:
(a) Zunächst wird der Stift 35 der Elektrode 1 beispielsweise mit einem Kunststoffmaterial umspritzt, welches dann die Aufnahmevorrichtung 23 bildet.
(b) Anschließend wird der nach außen ragende Stift 35 in geeigneter Weise umgebogen. Hierzu wird das nach außen ragende Ende des Stiftes 35 um den umspritzten Kunststoffkörper 23 so gebogen bzw. umschlauft, dass der Stift 35 einerseits fest fixiert ist und andererseits einen von außen elektrisch gut kontaktierbaren Kontakt ausbildet. Gegebenenfalls überstehende Enden des Kontaktstifts 35 werden abgeschnitten. Das nach innen gerichtete Ende des Stiftes 35 kann bei dieser Gelegenheit ebenfalls geeignet ausgerichtet werden.
(c) Anbringen ein es O-Ringes 24 an die Aufnahmevorrichtung 23.
(d) Separates Umspritzen der Ummantelung 11 des Messmoduls 10. Umspritzen des zweiten Stiftes 36 beispielsweise mit einem Kunststoffmaterial, welches die Bodenplatte 12 bildet. Der Kontaktstift, der die zweite Elektrode 15 bilden soll, wird an seinem äußeren Ende gebogen und in eine eigens in der Bodenplatte 12 vorgesehenen Öse 38 eingeschlauft. Mittels Ultraschallschweißen werden die Bodenplatte 12 und die Ummantelung 11 zur Bildung des Gehäuses 3 fest miteinander verbunden.
(e) Ein Glasröhrchen 33 wird bereit gestellt, in welches eine Elektrodenflüssigkeit eingefüllt wird. Anschließend wird das Glasröhrchen 33 in die Aufnahmevorrichtung 23 eingeklebt. Das Glasröhrchen 33 mit der darin enthaltenen Elektrodenflüssigkeit sowie der Kontaktstift 35 bilden die erste Elektrode 2.
(f) Schließlich wird diese erste Elektrode 2 samt Aufnahmevorrichtung 23 in die noch leere Kammer 14 des Gehäuses 3 eingeführt und geeignet fixiert. Eine Dichtvorrichtung (O-Ring 24) dichtet das Gehäuse 2 an der Seite der Bodenplatte 12 nach außen ab.

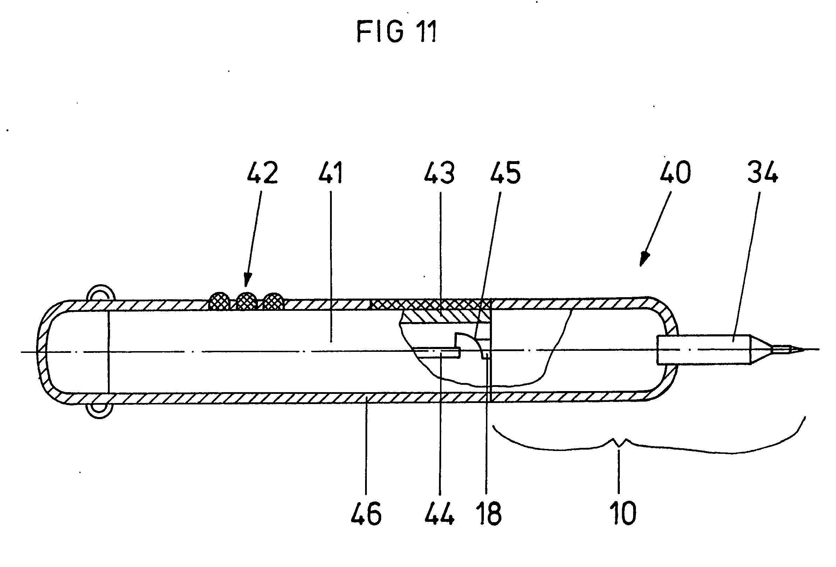

Figur 11 zeigt eine Querschnittsdarstellung eines mit Bezugszeichen 40 bezeichneten tragbaren Messgerätes. Das Messgerät 40 weist ein Gehäuse 41 auf, auf dem ein pH-Messmodul 10 aufgesteckt ist. Das pH-Messmodul 10 ist beispielsweise entsprechend den in den Figuren 2 bis 7 dargestellten Messmodulen ausgebildet und weist an der Unterseite der Bodenplatte 12 Kontaktflächen 18 auf.

Das Messgerät 40 weist eine Tastatur 42 zum Eingeben von Daten sowie ein Anzeigenfeld 43 zum Darstellen von Messergebnissen und Daten auf. Innerhalb des Gehäuses 41 ist eine Platine 44 angeordnet. Mittels Verbindungsleitungen, die hier als Federkontakte 45 ausgebildet sind, ist die Platine 44 mit den entsprechenden Kontaktflächen 18 des Messmoduls 10 kontaktierbar. Auf diese Weise ist das pH-Messmodul 10 mit dem Messgerät 40 elektrisch adaptiert.

Vorteilhafterweise ist das das Messmodul 10 aufweisende Messgerät 40 aus Stabilitäts-, Dichte- und Hygienegründen mit einer elastischen Schutzhülle 46, die zumindest im Bereich des Anzeigenfeldes 43 transparent oder zumindest teiltransparent ausgebildet ist, ausgestattet.

Zusammenfassend kann festgestellt werden, dass durch eine in ihrer axialen Richtung bewegliche Messelektrode diese auf sehr einfache, jedoch nichts desto Trotz sehr effektive Weise bei einem in Axialrichtung gerichteten Stoß gegen Zerstörung bzw. Beschädigung geschützt werden kann.

Die vorliegende Erfindung wurde anhand der vorstehenden Ausführungsbeispiele so dargestellt, um das Prinzip der Erfindung und dessen praktische Anwendung bestmöglichst darzulegen, jedoch lässt sich die Erfindung selbstverständlich bei geeigneter Abwandlung in mannigfaltigen anderen Ausführungsformen realisieren.

### Bezugszeichenliste

- 1: Messvorrichtung
- 2: erste Elektrode, Glaselektrode
- 3: Gehäuse des Messmoduls
- 4: Messspitze
- 5: Öffnung
- 6: oberes Ende des Gehäuses
- 7: Längsachse

- 10: (auswechselbares) Messmodul
- 11: Ummantelung
- 12: Bodenplatte, Trägerplatte
- 13: Ende der ersten Elektrode
- 14: Kammer
- 15: zweite Elektrode, Silberelektrode
- 16: Messgut
- 17: Aussparung
- 18: Kontaktflächen, Goldkontakte
- 19: Kontaktflächen, Goldkontakte
- 20: Boden der Aussparung
- 21: gefalzte Abschnitte
- 22: Feder
- 23: Aufnahmevorrichtung
- 24: O-Ring
- 25: Gummipuffer
- 26: Aussparung
- 27: Boden der Aussparung
- 28: umlaufende Dichtlippe
- 29: Seitenfläche der Aussparung
- 31: Steckverbindung
- 32: Glasröhrchen der ersten Elektrode
- 33: Schutzstege
- 34: Messsonde
- 35: (elektrisch leitfähige) Stifte
- 36: (elektrisch leitfähige) Stifte
- 37: Unterseite der Bodenplatte
- 38: Öse
- 39: Schweißnaht
- 40: (tragbares) Messgerät
- 41: Gehäuse des Messgerätes
- 42: Tastatur
- 43: Anzeigenfeld, Display
- 44: Platine
- 45: Federkontakte
- 46: Schutzhülle
- 47: Silberstift
- 48: oberes Ende der Aufnahmevorrichtung
- 49: unteres Ende der Aufnahmevorrichtung
- 50 .: Röhrchen mit Temperatursensor

- d1: Durchmesser der Messsonde
- X: Axialrichtung
- Y: Richtung senkrecht zur Axialrichtung
- F_{X}: Kraft in Axialrichtung

## Patentansprüche

1. Messvorrichtung, insbesondere pH-Messvorrichtung, mit einer langgestreckten, eine Längsachse (7) aufweisenden ersten Elektrode (2) zum Einstechen in ein Messgut (16) und mit einem die erste Elektrode (2) zumindest teilweise umgebenden Gehäuse (3), wobei eine von dem Gehäuse (3) umschlossene, nach außen hin dichte Kammer (14) vorgesehen ist, und wobei zwischen der ersten Elektrode (2) und einer Ummantelung (4) zumindest eine zweite Elektrode (15) angeordnet ist und dass eine Bodenplatte (12) einen unteren Bereich der Kammer (14) fest verschließt,
**dadurch gekennzeichnet,**
**dass** die ersten Elektrode (2) in Axialrichtung (X) ihrer Längsachse (7) beweglich ausgebildet ist.

2. Messvorrichtung nach Anspruch 1,
**dadurch gekennzeichnet,**
**dass** eine Aufnahmevorrichtung (17,23, 26) zur Aufnahme eines Endes (13) der ersten Elektrode (2) vorgesehen ist.

3. Messvorrichtung nach Anspruch 2,
**dadurch gekennzeichnet,**
**dass** die Aufnahmevorrichtung (17,23, 26) aus einem elastischen Material besteht, welches bei einer Bewegung der ersten Elektrode (2) in Axialrichtung (X) nachgibt.

4. Messvorrichtung nach einem der Ansprüche 2 oder 3,
**dadurch gekennzeichnet,**
**dass** die Aufnahmevorrichtung (17,23, 26) ein Dämpfungselement (22,25) aufweist oder mit einem Dämpfungselement verbunden ist, welches bei einer Bewegung der ersten Elektrode (2) in Axialrichtung (X) nachgibt und dabei eine in entgegengesetzte Richtung gerichtete Kraft auf das Ende (13) der ersten Elektrode (2) ausübt.

5. Messvorrichtung nach Anspruch 4,
**dadurch gekennzeichnet,**
**dass** das Dämpfungselement (22,25) als Gummipuffer (25) ausgebildet ist.

6. Messvorrichtung nach einem der Ansprüche 4 oder 5,
**dadurch gekennzeichnet,**
**dass** das Dämpfungselement (22,25) als Feder (22) ausgebildet ist.

7. Messvorrichtung nach einem der Ansprüche 4 bis 6,
**dadurch gekennzeichnet,**
**dass** das Gehäuse (3) auf der Seite der Aufnahmevorrichtung (17,23, 26) eine Bodenplatte (12) aufweist und die Aufnahmevorrichtung (17,23, 26) Bestandteil der Bodenplatte (12) ist.

8. Messvorrichtung nach Anspruch 7,
**dadurch gekennzeichnet,**
**dass** die Bodenplatte (12) aus einem elastischen Material besteht.

9. Messvorrichtung nach Anspruch 7 oder 8,
**dadurch gekennzeichnet,**
**dass** die Bodenplatte (12) membranartig ausgebildet ist und Falzabschnitte (21) aufweist.

10. Messvorrichtung nach der Ansprüche 2 bis 9,
**dadurch gekennzeichnet,**
**dass** die Aufnahmevorrichtung (17,23, 26) eine Aussparung (17,26) zur formschlüssigen Aufnahme des einen Endes (13) der ersten Elektrode (2) aufweist.

11. Messvorrichtung nach Anspruch 10,
**dadurch gekennzeichnet,**
**dass** eine Dichtvorrichtung (28) vorgesehen ist, über welche das eine Ende (13) der ersten Elektrode (2) fest in Anlage mit der Aussparung (17,26) gelangt.

12. Messvorrichtung nach einem der vorstehenden Ansprüche,
**dadurch gekennzeichnet,**
**dass** die Elektroden (2,15) am Gehäuse (3) angeklebt oder angeschweißt oder umspritzt sind.

13. Messvorrichtung nach einem der vorstehenden Ansprüche,
**dadurch gekennzeichnet,**
**dass** Kontaktstifte (35,36) vorgesehen sind, die mit den Elektroden (2,15) verbunden sind, die durch die Bodenplatte (12) hindurchragen und die an einer Unterseite (37) der Bodenplatte (12) derart abgebogen sind, dass das abgebogene Ende dieser Kontaktstifte (35,36) in eigens dafür vorgesehenen Ösen (38) an der Unterseite (37) der Bodenplatte (12) eingeschlauft sind.

14. Messvorrichtung nach der Anspruch 13,
**dadurch gekennzeichnet,**
**dass** in der Kammer (14) eine Polymerprotolyt-Flüssigkeit eingefüllt ist, die die erste und/oder die zweite Elektrode (2, 15) umgibt.

15. Messvorrichtung nach der vorstehenden Ansprüche,
**dadurch gekennzeichnet,**
**dass** die erste Elektrode (2,47) in einem Röhrchen (32), insbesondere einem Glasröhrchen (32), angeordnet ist, in welchem eine Elektrolyt-Flüssigkeit eingefüllt ist.

16. Messvorrichtung nach der Anspruch 15,
**dadurch gekennzeichnet,**
**dass** das Röhrchen (32) gegen die erste Elektrode (2) in Axialrichtung(X) verschiebbar ist.

17. Messvorrichtung nach der vorstehenden Ansprüche,
**dadurch gekennzeichnet,**
**dass** das Gehäuse (3) an einer Messspitze (4) verschiebbar gegenüber der ersten Elektrode (2) angeordnet ist.

18. Messvorrichtung nach der vorstehenden Ansprüche,
**dadurch gekennzeichnet,**
**dass** ein Durchmesser(dl) des Gehäuses (3) in Richtung zu einer Messspitze (4) der ersten Elektrode (2) abnimmt.

19. Messvorrichtung nach der vorstehenden Ansprüche,
**dadurch gekennzeichnet,**
**dass** die erste Elektrode (2) und/oder eine diese umschließende Schutzhülse (32) zumindest teilweise aus Glas besteht.

20. Messvorrichtung nach der vorstehenden Ansprüche,
**dadurch gekennzeichnet,**
**dass** die erste Elektrode (2) schwenkbar gelagert ist.

21. Messvorrichtung nach Anspruch 21,
**dadurch gekennzeichnet,**
**dass** die erste Elektrode (2) Schwenkmittel, insbesondere ein Gelenk, aufweist, mittel denen die erste Elektrode (2) im Falle einer Kraftkomponente (Fy) senkrecht zur Axialrichtung (X) von der Axialrichtung (X) weg schwenkbar ist.

22. Messvorrichtung nach der vorstehenden Ansprüche,
**dadurch gekennzeichnet,**
**dass** das Gehäuse (3) zumindest zum Teil einen Werkstoff aus SAN oder ABS enthält.

23. Tragbares pH-Messgerät (40) mit einer modularen, auswechselbaren pH-Messvorrichtung (1, 10) nach einem der vorstehenden Ansprüche.

24. PH-Messgerät nach Anspruch 23,
**gekennzeichnet durch**
ein Gehäuse (41), in dem ein Anzeigenfeld (43) und eine Tastatur (42) angeordnet sind, und **durch** eine Platine (44), von der Federkontakte (45) zu an einer Unterseite (37) der Bodenplatte (12) angeordneten Kontakten (18,19) der ersten und zweiten Elektroden (2,15) wegführen.

25. Verfahren zur Herstellung einer Messvorrichtung nach einem der Ansprüche 1 bis 22,
mit den folgenden Montageschritten:
(a) Umspritzen von elektrisch leitenden Kontaktstiften (35, 36) zur Bildung einer Bodenplatte (12), aus der die Kontaktstiften (35,36) für eine erste und/oder für eine zweite Elektrode (1, 15) herausragen;,
(b) Einfüllen einer Elektrodenflüssigkeit in ein Röhrchen (32);
(c) Einschieben der ersten Elektrode in das Röhrchen (32) und Verkleben des Röhrchens (32) mit der Bodenplatte (12);
(d) Verschweißen der Bodenplatte (12) mit einer leeren Ummantelung (11) zur Bildung eines Gehäuses (3), welches eine Kammer (14) aufweist;
(e) Einfüllen einer Polymerprotolyt-Flüssigkeit der zweiten Elektrode (15) in die Kammer (14).

26. Verfahren nach Anspruch 25,
**dadurch gekennzeichnet,**
**dass** die aus dem Gehäuse (3) herausragenden Kontaktstifte (35,36) zur Bildung von Kontakten an der Außenwand des Gehäuses (3) umgebogen werden.

27. Verwendung einer Messvorrichtung nach einem der Ansprüche 1 bis 22 oder eines Messgerätes nach einem der Ansprüche 23 oder 24 zur Messung des pH-Wertes in Lebensmitteln, von eiweißhaltigen Flüssigkeiten oder Abwässern, wobei die Elektroden (2,15) von einer Polymerprotolyt-Substanz umgeben sind.

## Claims

1. Measuring device, in particular a pH meter, with an elongated first electrode (2), which has a longitudinal axis (7), for insertion into a material to be measured (16), and with a housing (3) that at least partially surrounds the first electrode (2), wherein a chamber (14) is provided which is enclosed by the housing (3) and which is tight relative to the outside, and wherein arranged between the first electrode (2) and a casing (4) there is at least one second electrode (15), and that a base plate (12) tightly seals a lower area of the chamber (14),
**characterised in that**
the first electrode (2) is designed to be movable in the axial direction (X) of its longitudinal axis (7).

2. Measuring device according to claim 1, **characterised in that** a receptacle device (17, 23, 26) is provided, to accommodate one end (13) of the first electrode (2).

3. Measuring device according to claim 2, **characterised in that** the receptacle device (17, 23, 26) consists of an elastic material, which yields in the axial direction (X) in the event of a movement of the first electrode (2).

4. Measuring device according to one of the claims 2 or 3, **characterised in that** the receptacle device (17, 23, 26) has a damping element (22, 25) or is connected to a damping element, which in the event of a movement of the first electrode (2) yields in the axial direction (X), and in so doing exerts a force directed in the opposite direction on the end (13) of the first electrode (2).

5. Measuring device according to claim 4, **characterised in that** the damping element (22, 25) is designed as a rubber buffer (25).

6. Measuring device according to one of the claims 4 or 5, **characterised in that** the damping element (22, 25) is designed as a spring (22).

7. Measuring device according to one of the claims 4 to 6, **characterised in that** the housing (3) has a base plate (12) on the side of the receptacle device (17, 23, 26), and the receptacle device (17, 23, 26) is a constituent part of the base plate (12).

8. Measuring device according to claim 7, **characterised in that** the base plate (12) consists of an elastic material.

9. Measuring device according to claim 7 or 8, **characterised in that** the base plate (12) is designed in the manner of a diaphragm, and has folded sections (21).

10. Measuring device according to the claims 2 to 9, **characterised in that** the receptacle device (17, 23, 26) has a recess (17, 26) to accommodate one end (13) of the first electrode (2) in a form-fitting manner.

11. Measuring device according to claim 10, **characterised in that** a sealing device (28) is provided, via which one end (13) of the first electrode (2) comes to rest firmly with the recess (17, 26).

12. Measuring device according to one of the preceding claims, **characterised in that** the electrodes (2, 15) are glued to the housing (3) or welded to it or injection moulded.

13. Measuring device according to one of the preceding claims, **characterised in that** contact pins (35, 36) are provided which are connected to the electrodes (2, 15) which protrude through the base plate (12) and which on an underside (37) of the base plate (12) are bent in such a way that the bent ends of these contact pins (35, 36) are threaded into eyes (38) on the underside (37) of the base plate (12) that are provided specifically for that purpose.

14. Measuring device according to claim 13, **characterised in that** a polymer protolyte fluid is filled into the chamber (14), and surrounds the first and/or second electrode (2, 15).

15. Measuring device according to the preceding claims, **characterised in that** the first electrode (2, 47) is arranged in a tube (32), in particular a glass tube (32), into which an electrolyte fluid is filled.

16. Measuring device according to claim 15, **characterised in that** the tube (32) can be moved in an axial direction (X) against the first electrode (2).

17. Measuring device according to the preceding claims, **characterised in that** the housing (3) is arranged so as to be movable on a measuring probe (4) relative to the first electrode (2).

18. Measuring device according to the preceding claims, **characterised in that** one diameter (dl) of the housing (3) decreases in the direction towards a measuring probe (4) of the first electrode (2).

19. Measuring device according to the preceding claims, **characterised in that** the first electrode (2) and/or a protective sleeve (32) enclosing it consists at least partially of glass.

20. Measuring device according to the preceding claims, **characterised in that** the first electrode (2) is mounted such that it can be pivoted.

21. Measuring device according to claim 21 [*sic*], **characterised in that** the first electrode (2) has pivoting means, in particular a joint, by means of which in the case of a force component (Fy) perpendicular to the axial direction (X), the first electrode (2) can be pivoted away from the axial direction (X).

22. Measuring device according to the preceding claims, **characterised in that** the housing (3) at least in part contains a material of SAN or ABS.

23. Portable pH meter (40) with a modular, replaceable pH meter (1, 10) according to one of the preceding claims.

24. PH meter according to claim 23, **characterised by** a housing (41), arranged in which are a display field (43) and a keyboard (42), and by a circuit board (44) from which spring contacts (45) lead away to contacts (18, 19) of the first and second electrodes (2, 15), these contacts being arranged on an underside (37) of the base plate (12).

25. Method for producing a measuring device according to one of the claims 1-22, with the following assembly steps:
(a) injection-moulding around electrically conductive contact pins (35, 36) to form a base plate (12), from which the contact pins (35, 36) for a first and/or a second electrode (1, 15) project;
(b) filling an electrode fluid into a tube (32);
(c) pushing the first electrode into the tube (32) and gluing the tube (32) to the base plate (12) ;
(d) welding of the base plate (12) to an empty casing (11) to form a housing (3) that has a chamber (14);
(e) filling a polymer protolyte fluid of the second electrode (15) into the chamber (14).

26. Method according to claim 25, **characterised in that** the contact pins (35, 36) protruding out of the housing (3) are bent over to form contacts on the outer wall of the housing (3).

27. Use of a measuring device according to one of the claims 1 to 22, or of a measuring device according to one of the claims 23 or 24, for measuring the pH value in foodstuffs, protein-containing liquids or waste water, wherein the electrodes (2, 15) are surrounded by a polymer protolyte substance.

## Revendications

1. Dispositif de mesure notamment dispositif de mesure de pH, comportant une première électrode (2), allongée, ayant un axe longitudinal (7) pour être enfoncée dans un produit à mesurer (16) et un boîtier (3) entourant au moins partiellement la première électrode (2), le boîtier (3) entourant une chambre (14) de manière étanche vis-à-vis de l'extérieur, et
au moins une seconde électrode (15) est prévue entre la première électrode (2) et une enveloppe (11), et
une plaque de fond (12) ferme de manière solidaire la zone inférieure de la chambre (14),
**caractérisé en ce que**
la première électrode (2) est mobile dans la direction axiale (X) de son axe longitudinal (7).

2. Dispositif de mesure selon la revendication 1,
**caractérisé par**
un dispositif de réception (17, 23, 26) pour recevoir une extrémité (13) de la première électrode (2).

3. Dispositif de mesure selon la revendication 2,
**caractérisé en ce que**
le dispositif de réception (17, 23, 26) est en une matière élastique qui est souple pour un mouvement de la première électrode (2) dans la direction axiale (X).

4. Dispositif de mesure selon la revendication 2 ou 3,
**caractérisé en ce que**
le dispositif de réception (17, 23, 26) comporte un élément amortisseur (22, 25) ou est relié à un élément amortisseur qui cède dans la direction axiale (X) sous l'effet d'un mouvement de la première électrode (2), et exerce ainsi une force dirigée dans la direction opposée sur l'extrémité (13) de la première électrode (2).

5. Dispositif de mesure selon la revendication 4,
**caractérisé en ce que**
l'élément amortisseur (22, 25) est un tampon en caoutchouc (25).

6. Dispositif de mesure selon la revendication 4 ou 5,
**caractérisé en ce que**
l'élément amortisseur (22, 25) est en forme de ressort (22).

7. Dispositif de mesure selon les revendications 4 à 6,
**caractérisé en ce que**
le boîtier (3) comporte une plaque de fond (12) sur le côté du dispositif de réception (17, 23, 26) et ce dispositif de réception (17, 23, 26) fait partie de la plaque de fond (12).

8. Dispositif de mesure selon la revendication 7,
**caractérisé en ce que**
la plaque de fond (12) est en une matière élastique.

9. Dispositif de mesure selon la revendication 7 ou 8,
**caractérisé en ce que**
la plaque de fond (12) est en forme de membrane et comporte des segments de pli (21).

10. Dispositif de mesure selon les revendications 2 à 9,
**caractérisé en ce que**
le dispositif de réception (17, 23, 26) comporte une cavité (17, 26) pour recevoir par une liaison de forme l'extrémité (13) de la première électrode (2).

11. Dispositif de mesure selon la revendication 10,
**caractérisé par**
un dispositif d'étanchéité (28) par lequel l'extrémité (13) de la première électrode (2) vient fermement en appui dans la cavité (17, 26).

12. Dispositif de mesure selon les revendications précédentes,
**caractérisé en ce que**
les électrodes (2, 15) sont collées, soudées ou surmoulées dans le boîtier (3).

13. Dispositif de mesure selon les revendications précédentes,
**caractérisé par**
des broches de contact (35, 36) reliées aux électrodes (2, 15) et traversant la plaque de fond (12) pour être recourbées contre la face inférieure (37) de la plaque de fond (12) pour que l'extrémité recourbée des broches de contact (35, 36) puisse être engagée dans des oeillets (38) prévus à cet effet sur la face inférieure (37) de la plaque de fond (12).

14. Dispositif de mesure selon la revendication 13,
**caractérisé en ce que**
la chambre (14) contient un liquide de polymère protolythe, entourant la première et/ou la seconde électrode (2, 15).

15. Dispositif de mesure selon les revendications précédentes,
**caractérisé en ce que**
la première électrode (2, 47) est placée dans un petit tube (32) notamment un petit tube en verre (32) chargé d'un électrolyte liquide.

16. Dispositif de mesure selon la revendication 15,
**caractérisé en ce que**
le petit tube (32) peut coulisser dans la direction axiale (X) par rapport à la première électrode (2).

17. Dispositif de mesure selon les revendications précédentes,
**caractérisé en ce que**
le boîtier (3) est coulissant par rapport à la première électrode (2) au niveau de la pointe de mesure (4).

18. Dispositif de mesure selon les revendications précédentes,
**caractérisé en ce que**
le diamètre (d1) du boîtier (3) diminue en direction de la pointe de mesure (4) de la première électrode (2).

19. Dispositif de mesure selon les revendications précédentes,
**caractérisé en ce que**
la première électrode (2) et/ou un manchon protecteur (32) entourant celle-ci sont au moins en partie en verre.

20. Dispositif de mesure selon les revendications précédentes,
**caractérisé en ce que**
la première électrode (2) est pivotante.

21. Dispositif de mesure selon la revendication 20,
**caractérisé en ce que**
la première électrode (2) comporte un moyen de pivotement notamment une articulation à l'aide de laquelle la première électrode (2) peut s'écarter de la direction axiale (X) perpendiculairement à cette direction axiale (X) au cas où elle est soumise à une composante de force (Fy).

22. Dispositif de mesure selon les revendications précédentes,
**caractérisé en ce que**
le boîtier (3) comporte au moins en partie un matériau tel que SAN ou ABS.

23. Appareil de mesure de pH, portatif (40) comportant un dispositif de mesure de pH (11, 10) modulaire, interchangeable selon les revendications précédentes.

24. Appareil de mesure de pH selon la revendication 23.
**caractérisé par**
un boîtier (41) logeant un afficheur (43) et un clavier (42), et une platine (44) d'où partent des contacts élastiques (45) reliés à des contacts (18, 19) prévus sur la face inférieure (37) de la plaque de fond (12), contacts correspondant à la première et à la seconde électrodes (2, 15).

25. Procédé de fabrication d'un dispositif de mesure selon les revendications 1 à 22, comprenant les étapes de montage suivantes:
(a) Surmoulage par injection de broches de contact (35, 36) électrique pour former une plaque de fond (12) d'où les broches de contact (35, 36) sont en saillie pour une première et/ou une seconde électrodes (2, 15),
(b) Introduction d'un liquide d'électrode dans un petit tube (32),
(c) Engagement de la première électrode dans le petit tube (32) et collage du petit tube (32) à la plaque de fond (12),
(d) Soudage de la plaque de fond (12) à une enveloppe vide (11) pour former un boîtier (3) muni d'une chambre (14),
(e) Introduction d'un liquide protolythe polymère de la seconde électrode dans la chambre (14).

26. Procédé de fabrication selon la revendication 25,
**caractérisé en ce que**
les broches de contact (35, 36) qui dépassent du boîtier (3) sont recourbées pour former les contacts sur la paroi extérieure du boîtier (3).

27. Utilisation d'un dispositif de mesure selon les revendications 1 à 22 ou d'un appareil de mesure selon la revendication 23 ou 24 pour mesurer le pH dans des produits alimentaires, de liquide contenant de l'albumine ou des eaux usées, les électrodes (2, 15) étant entourées d'une substance protolythe polymère.
